# EUROPÄISCHE PATENTANMELDUNG

(11) **EP 3 816 174 A1**
(43) Veröffentlichungstag der Anmeldung: **05.05.2021**
(21) Anmeldenummer: 19205658.8
(22) Anmeldetag: 28.10.2019
(51) Int. Cl.: C07F 9/6574, C07C 45/50, C07F 15/00

(54) **BENZOPINACOLPHOSPHITE**

(71) Anmelder: Evonik Operations GmbH, 45128 Essen (DE)
(72) Erfinder: FRANKE, Robert, 45772 Marl (DE); BÖRNER Armin, 18059 Rostock (DE); SELENT, Detlef, 18059 Rostock (DE); KLOSS, Svenja, 18057 Rostock (DE)
(74) Vertreter: Evonik Patent Association

(57) **Zusammenfassung**

Benzopinacolphosphite und deren Verwendung in der Hydroformylierung.

## Beschreibung

Die Erfindung betrifft Benzopinacolphosphite und deren Verwendung in der Hydroformylierung.

Phosphorhaltige Verbindungen spielen als Liganden in einer Vielzahl von Reaktionen eine entscheidende Rolle, z.B. in der Hydrierung, in der Hydrocyanierung und auch in der Hydroformylierung.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803.

Die technische Aufgabe der Erfindung ist die Bereitstellung neuer Liganden, welche in der Hydroformylierung von Olefinen eine gesteigerte n-Regioselektivität aufweisen.

Die Aufgabe wird gelöst durch eine Verbindung gemäß Anspruch 1.

Verbindung gemäß der Struktur (**1**) oder (**2**):

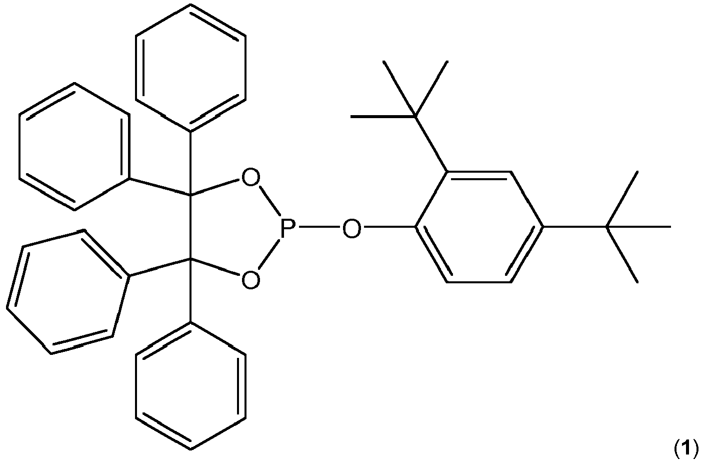

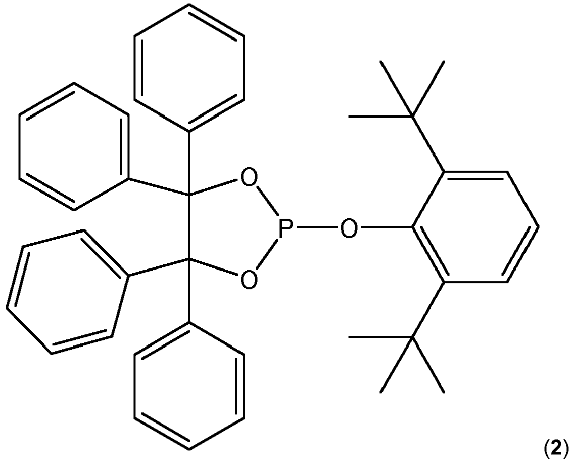

In einer Ausführungsform weist die Verbindung die Struktur (**1**) auf:

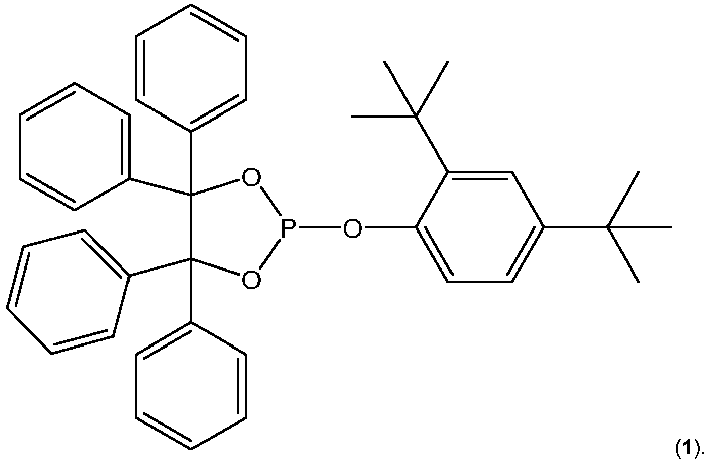

In einer Ausführungsform weist die Verbindung die Struktur (**2**) auf:

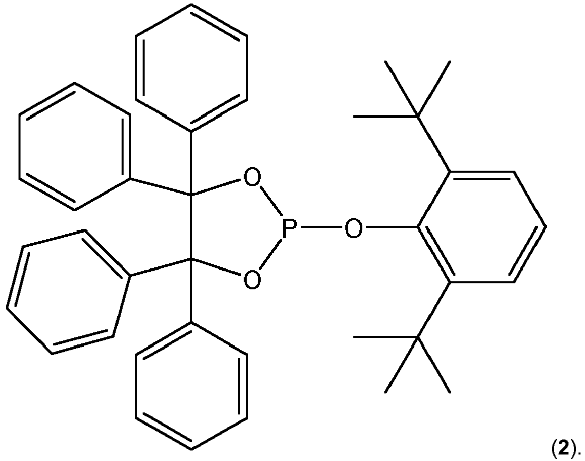

Neben der Verbindung als solche wird auch deren Verwendung zur Katalyse einer Hydroformylierungsreaktion beansprucht.

Verwendung einer zuvor beschriebenen Verbindung in einem Ligand-Metall-Komplex zur Katalyse einer Hydroformylierungsreaktion.

Des Weiteren wird ein Verfahren beansprucht, in welchem die zuvor beschriebene Verbindung als Ligand eingesetzt wird.

Verfahren umfassend die Verfahrensschritte:
a) Vorlegen eines Olefins,
b) Zugabe einer zuvor beschriebenen Verbindung und einer Substanz, welche ein Metall ausgewählt aus: Rh, Ru, Co, Ir aufweist,
c) Zuführen von H₂ und CO,
d) Erwärmen des Reaktionsgemisches aus a) bis c), wobei das Olefin zu einem Aldehyd umgesetzt wird.

In einer bevorzugten Ausführungsform ist das Metall Rh.

Es kann hierbei auch ein Überschuss an Liganden verwendet werden und nicht zwangsläufig jeder Ligand liegt gebunden in Form eines Ligand-Metall-Komplexes vor, sondern ist als freier Ligand im Reaktionsgemisch enthalten.

Die Reaktion wird bei üblichen Bedingungen durchgeführt.

Bevorzugt sind eine Temperatur von 80 °C bis 160 °C und ein Druck von 10 bis 70 bar. Besonders bevorzugt sind eine Temperatur von 100 °C bis 140 °C und ein Druck von 40 bis 60 bar.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1-Buten, 2-Buten, 1- oder 2-Penten, 2-Methyl-1-buten, 2-Methyl-2-buten, 3-Methyl-1-buten, 1-, 2- oder 3,-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Di-n-buten, Di-iso-buten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung von Propen oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabuten) sowie durch Cooligomerisierung von Olefinen mit unterschiedlicher Anzahl von Kohlenstoffatomen (bevorzugt 2 bis 4) hergestellte Olefingemische.

Mit dem erfindungsgemäßen Verfahren können unter Verwendung der erfindungsgemäßen Liganden α-Olefine, endständig verzweigte, innenständige und innenständig verzweigte Olefine hydroformyliert werden.

Im Folgenden soll die Erfindung anhand von Ausführungsbeispielen näher erläutert werden.

### Arbeitsvorschriften

### Allgemeine Analytik

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet.

Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR³¹P = SR¹H * (BF³¹P / BF¹H) = SR¹H * 0,4048.

### Synthese 2-(2,4-Di-tert-butylphenoxy)-4,4,5,5-tetraphenyl-1,3,2-dioxaphospholan (1)

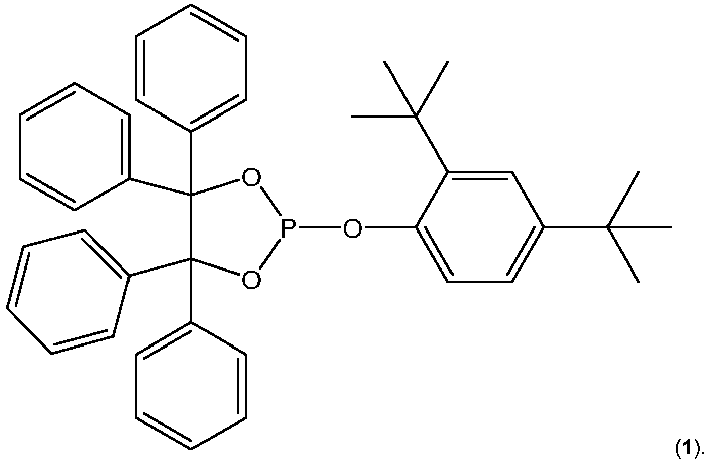

Zu einer gerührten Suspension von 0,247 g 2,4-Di-*tert*-butylphenol (1,201 mmol) und 5 ml Toluol wurden bei 0 °C 0,4 ml Triethylamin (2,97 mmol) getropft. Anschließend wurde eine Lösung aus 0,52 g 2-Chloro-4,4,5,5-tetraphenyl-1,3,2-dioxaphospholan (1,201 mmol) in 5 ml Toluol tropfenweise hinzugegeben. Die Reaktionslösung wurde für 24 h bei Raumtemperatur gerührt und dann filtriert. Das Filtrat wurde im Vakuum bis zur Trockene eingeengt. Der Rückstand wurde erneut in 15 ml Toluol gelöst und durch eine dünne Schicht Silica filtriert. Das Lösungsmittel wurde im Vakuum entfernt und das feste Produkt bei 0,1 bar bei 40 °C für 5 h getrocknet. Ausbeute: 0,395 g (55%).
¹H-NMR (300 MHz, CD₂Cl₂): δ (ppm) = 1,25 (s; 9H; 3CH₃); 1,32 (s; 9H; 3CH₃); 6,90 (d; *J*_{HH}= 8,18 Hz); 7,05-7,20 (m; 17H); 7,36 (d; *J*_{HH}= 2,24 Hz; 1H); 7,56-7,59 (m; 4H).
¹³C-NMR (75 MHz, CD₂Cl₂): δ (ppm) = 30,4 (3CH₃); 31,8 (3CH₃); 34,9 (C); 35,1 (C); 95,8 (d; *J*_{CP}= 8,2 Hz); 121,7 (d; *J*_{CP}= 15,3 Hz); 124,0; 124,8; 127,6; 127,7; 127,8; 129,3 (d; *J*_{CP}= 3,4 Hz); 130,3; 140,7 (d; *J*_{CP}= 2,6 Hz); 142,6 (d; *J*_{CP} = 4,3 Hz); 143,2; 146,9; 148,4 (d; *J*_{CP}= 10,0 Hz). ³¹P-NMR (121 MHz, CD₂Cl₂): δ (ppm) = 138,5 (s).
HRMS (ESI): Berechnet für C₄₀H₄₁O₃P (M+H)⁺; 601,2866 gefunden 601,2866.

### Synthese 2-(2,6-Di-tert-butylphenoxy)-4,4,5,5-tetraphenyl-1,3,2-dioxaphospholan (2)

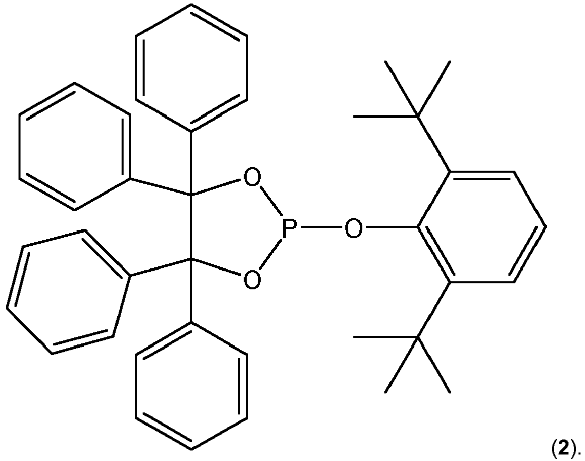

Zu einer gerührten Suspension von 0,41 g 2,6-Di-*tert*-butylphenol (2,00 mmol) in 5 ml THF wurden bei 0 °C 1,00 ml einer n-BuLi-Lösung (2,5 M in Hexan; 2,50 mmol) getropft. Die Lösung wurde für 20 min weiter gerührt und langsam auf Raumtemperatur erwärmt. Anschließend wurde eine kalte Lösung von 0,86 g 2-Chloro-4,4,5,5-tetraphenyl-1,3,2-dioxaphospholan (2,00 mmol) in 5 ml THF tropfenweise hinzugegeben. Die Reaktionslösung wurde für weitere 24 h bei Raumtemperatur gerührt und anschließend filtriert. Das Filtrat wurde im Vakuum zur Trockene eingeengt. Der Rückstand wurde in 15 ml Toluol gelöst, die Lösung durch eine dünne Schicht Silica filtriert und das Lösungsmittel im Vakuum entfernt. Das feste Produkt wurde dann bei 0,1 bar bei 40 °C für 5 h getrocknet. Ausbeute: 0,853 g (1,42 mmol, 71%).
¹H-NMR (300 MHz, CD₂Cl₂): δ (ppm) = 1,36 (s; 18H; 9CH₃); 6,97-7,22 (m; 18H); 7,32 (d; *J*_{HH} = 8,31 Hz; 2H); 7,62-7,67 (m; 3H).
¹³C-NMR (75 MHz, CD₂Cl₂): δ (ppm) = 31,9 (3CH₃); 32,0 (3CH₃); 35,5; 96,2 (d; *J*_{CP} = 9,0 Hz); 124,2; 126,7; 127,6 (d; *J*_{CP} = 23,6 Hz); 127,7 (d; *J*_{CP} = 34,4 Hz); 129,2 (d; *J*_{CP} = 4,3 Hz); 130,8; 142,4 (d; *J*_{CP} = 4,8 Hz); 142,7; 144,3 (d; *J*_{CP} = 3,2 Hz); 158,8 (d; *J*_{CP} = 11,8 Hz).
³¹P-NMR (121 MHz, CD₂Cl₂): δ (ppm) = 145,6 (s).
HRMS (ESI): Berechnet für C₄₀H₄₁O₃P (M+H)⁺; 601,2856 gefunden 601,2866.

### Synthese 2-(4-(tert-Butyl)phenoxy)-4,4,5,5-tetraphenyl-1,3,2-dioxaphospholan (3) (Vergleichsligand)

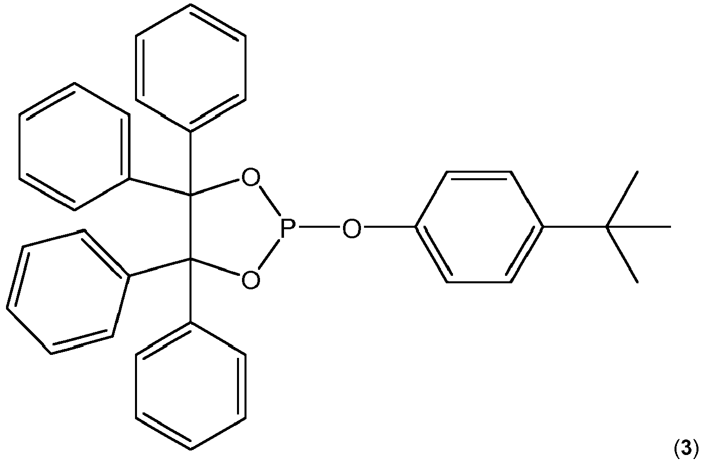

Zu einer gerührten Suspension aus 0,18 g 4-(*tert*-Butyl)phenol (1,20 mmol) in 5 ml Toluol wurden bei Raumtemperatur 4 ml Triethylamin (2,97 mmol) getropft. Anschließend wurde zu dieser Lösung eine kalte Lösung aus 0,52 g 2-Chloro-4,4,5,5-tetraphenyl-1,3,2-dioxaphospholan (1,20 mmol) in Toluen (5 ml) getropft. Die Reaktionslösung wurde für 24 h bei Raumtemperatur gerührt und filtriert. Das Filtrat wird im Vakuum bis zur Trockene eingeengt. Der erhaltene Rückstand wurde in 15 ml Toluol aufgenommen und durch eine dünne Schicht Silica filtriert. Das Lösungsmittel wurde im Vakuum entfernt, das feste Produkt bei 0,1 bar und 40 °C für 5 h getrocknet und so 0,61 g eines weißes Feststoffes erhalten (Ausbeute 93%).
¹H-NMR (300 MHz, CD₂Cl₂): δ (ppm) = 1,29 (s; 9H; 3CH₃); 6,65-6,71 (m; 2H); 7,05-7,15 (m; 9H); 7,19-7,29 (m; 9H); 7,56-7,61 (m; 4H).
¹³C-NMR (75 MHz, CD₂Cl₂): δ (ppm) = 31,2 (3CH₃); 34,2 (C); 95,2 (d; *J*_{CP} = 7,7 Hz); 120,1(d; *J*_{CP}= 7,7 Hz); 126,5; 127,2 (d; *J*_{CP} = 18,9 Hz); 127,3 (d; *J*_{CP} = 18,9 Hz); 128,8 (d; *J*_{CP} = 3,5 Hz); 129,9; 142,6 (d; *J*_{CP} = 4,0 Hz); 142,7; 146,9; 148,6 (d; *J*_{CP}= 8,7 Hz) ppm.
³¹P-NMR (121 MHz, CD₂Cl₂): δ (ppm) = 139,2 (s) ppm.

### Katalyseversuche

Die Hydroformylierung wurde in einem mit Druckkonstanthaltung, Gasflussmessung, und Begasungsrührer ausgestatteten 16 ml-Autoklaven der HEL group, Hertfordshire, Großbritannien, durchgeführt. Das als Substrat eingesetzte n-Octen (Oxeno GmbH, Octenisomerengemisch aus 1-Octen: 3,3 %; cis+trans-2-Octen: 48,5 %; cis+trans-3-Octen: 29,2 %; cis+trans-4-Octen: 16,4 %; gerüstisomere Octene: 2,6 %) wurde mehrere Stunden über Natrium am Rückfluss erhitzt und unter Argon destilliert.

Für die Versuche wurden die Reaktionslösungen vorab unter Argonatmosphäre vorbereitet. Hierfür wurden 0,0021 g Rh(acac)(CO)₂ und die entsprechende Menge an Phosphitverbindung eingewogen und mit 8,0 ml Toluol aufgefüllt. Die jeweils eingebrachte Masse an Toluol wurde für die GC-Analyse bestimmt. Anschließend wurden 1,80 g n-Octen (16 mmol) hinzugegeben. Die vorbereiteten Lösungen wurden anschließend in den Autoklaven eingefüllt und dieser dreimal mit Argon und dreimal mit Synthesegas (Linde; H₂ (99,999 %): CO (99,997%) = 1:1) gespült. Dann wurde der Autoklav bei einem Gesamtdruck von 10 bar unter Rühren (900 U/min) auf die angestrebte Temperatur aufgeheizt. Nach Erreichen der Reaktionstemperatur wurde der Synthesegasdruck auf 50 bar erhöht und die Reaktion bei konstantem Druck für 4 h geführt. Der Autoklav wurde nach Ablauf der Reaktionszeit auf Raumtemperatur abgekühlt, unter Rühren entspannt und mit Argon gespült. Jeweils 0,5 ml der Reaktionsmischungen wurden nach Beenden der Reaktion entnommen, mit 4 ml Pentan verdünnt und gaschromatographisch analysiert: HP 5890 Series II plus, PONA, 50 m x 0,2 mm x 0,5 µm. Die quantitative Bestimmung von Restolefin und Aldehyd erfolgte gegen das Lösungsmittel Toluol als interner Standard.

### Ergebnisse der Katalyseversuche

[Rh]: 1,0*10⁻³ mol/l, L/Rh: 5, p: 50 bar, T: 120 °C; t: 4 h

**Tabelle: Hydroformylierung der n-Octene**

| Eintrag | Ligand | Regioselektivität in % |
|---|---|---|
| 1 | **1*** | 31,1 |
| 2 | **2*** | 23,6 |
| 3 | **3** | 21,5 |

| | | |
|---|---|---|
| * erfindungsgemäße Verbindung | | |

### Definition der Selektivität:

Bei der Hydroformylierung gibt es die n/iso-Selektivitäten: das Verhältnis von linearem Aldehyd (= n) zu verzweigtem Aldehyd (= iso). Hierbei bedeutet die Regioselektivität bezüglich des n-Aldehyden, dass diese Menge an linearem Produkt gebildet wurde. Die restlichen Prozente entsprechen dann dem verzweigten Isomer. Bei einer Regioselektivität von 50% entstehen also n-Aldehyd und iso-Aldehyd zu gleichen Teilen.

Mit den erfindungsgemäßen Verbindungen (**1**) und (**2**) konnte jeweils eine gegenüber dem Vergleichsliganden (**3**) gesteigerte Regioselektivität erzielt werden.

Die durchgeführten Versuche belegen, dass die gestellte Aufgabe durch die erfindungsgemäßen Verbindungen gelöst wird.

## Patentansprüche

1. Verbindung gemäß der Struktur (**1**) oder (**2**):

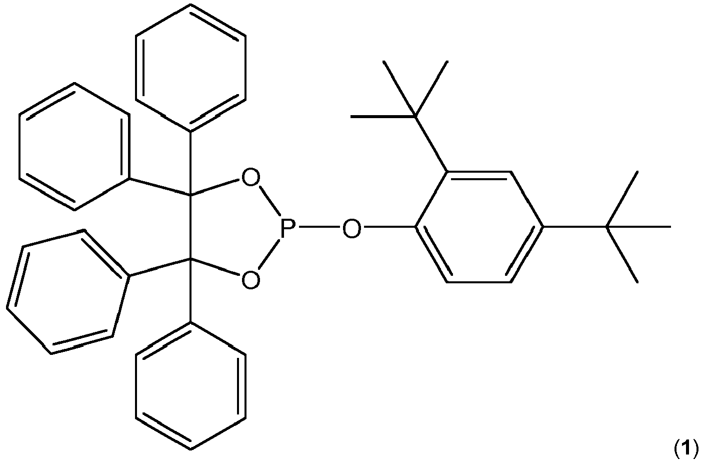

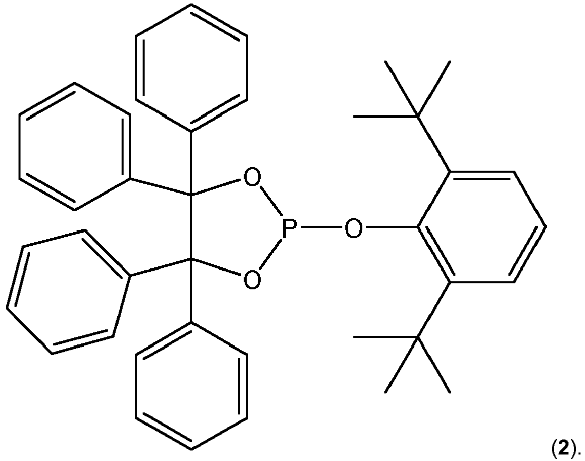

2. Verbindung nach Anspruch 1,
wobei die Verbindung die Struktur (1) aufweist:

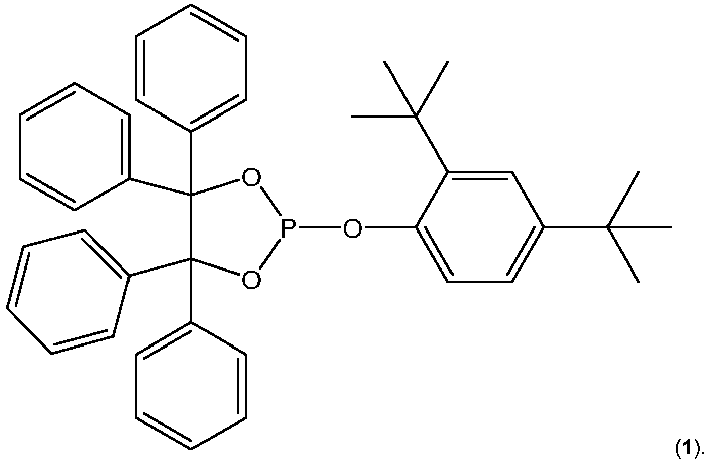

3. Verbindung nach Anspruch 1,
wobei die Verbindung die Struktur (**2**) aufweist:

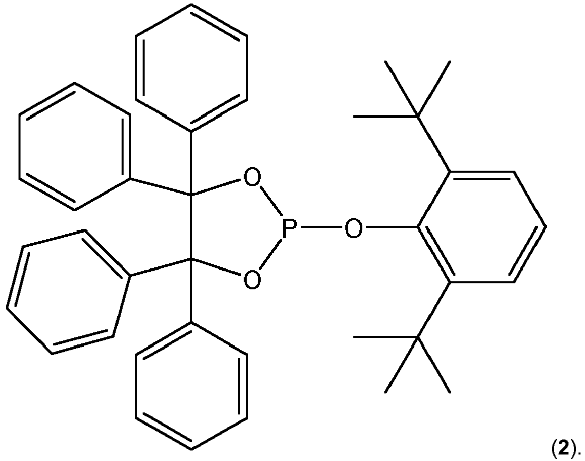

4. Verwendung einer Verbindung nach einem der Ansprüche 1 bis 3,
in einem Ligand-Metall-Komplex zur Katalyse einer Hydroformylierungsreaktion.

5. Verfahren umfassend die Verfahrensschritte:
a) Vorlegen eines Olefins,
b) Zugabe einer Verbindung nach einem der Ansprüche 1 bis 3 und einer Substanz, welche ein Metall ausgewählt aus: Rh, Ru, Co, Ir aufweist,
c) Zuführen von H₂ und CO,
d) Erwärmen des Reaktionsgemisches aus a) bis c), wobei das Olefin zu einem Aldehyd umgesetzt wird.
